# EUROPEAN PATENT APPLICATION

(11) **EP 4 245 854 A1**
(43) Date of publication of application: **20.09.2023**
(21) Application number: 21891887.8
(22) Date of filing: 10.11.2021
(51) Int. Cl.: C12N 15/113, A61K 31/7105, A61K 31/7115, A61K 31/712, A61P 1/16

(54) **MIR-29A-LIKE NUCLEIC ACID REAGENT, LIVER FIBROSIS INHIBITOR, AND PHARMACEUTICAL COMPOSITION**

(30) Priority: 10.11.2020 JP 2020187521
(71) Applicant: TOKYO MEDICAL UNIVERSITY, Tokyo 160-8402 (JP)
(72) Inventor: MURAKAMI Yoshiki, Tokyo 160-8402 (JP); KURODA Masahiko, Tokyo 160-8402 (JP)
(74) Representative: Dehns
(86) International application number: PCT/JP2021/041282
(87) International publication number: WO 2022/102641

(57) **Abstract**

Provided are an miR-29a-like nucleic acid reagent and a pharmaceutical composition that aim to inhibit liver fibrosis. An miR-29alike nucleic acid reagent according to the present invention includes nucleic acid having a base sequence of Sequence ID No. 1 that includes DNA and RNA and in which at least one nucleotide residue is a modified residue: Sequence ID No. 1: uagcaccaggctgucc. A liver fibrosis inhibitor and a pharmaceutical composition according to the present invention include the miR-29a-like nucleic acid reagent according to the present invention, and are preferably used for oral administration.

## Description

### Technical Field

The present invention relates to an miR-29a-like nucleic acid reagent, a liver fibrosis inhibitor, and a pharmaceutical composition.

### Background Art

It is known that, if chronic liver diseases are not appropriately treated, liver fibrosis may develop into liver cirrhosis. Chronic liver diseases are caused by various diseases such as chronic infection with HBV, HCV, and the like as well as alcohol-ingestion-induced diseases, fatty liver (particularly non-alcoholic steatohepatitis), autoimmune hepatitis, primary biliary cholangitis, and the like. Nowadays, there are no drugs that directly act to improve or prevent liver fibrosis, and therefore, a useful therapeutic method is liver transplantation in the case where the liver functions are significantly impaired due to liver cirrhosis, followed by liver failure. It is also known that the development of liver cirrhosis leads to an 8% annual rate of increase in the development of cancer. Accordingly, the treatment of liver fibrosis is extremely important.

In recent years, nucleic acid medicines have attracted attention as a novel therapeutic method. It has also been reported that excessive administration of miR-29a, which is micro-RNA, in treatment of liver fibrosis exhibits an effect of improving liver fibrosis (Non-Patent Document 1).

### Citation List

### Non-Patent Document

Non-Patent Document 1: Matsumoto Y, et al. MiR-29a assists in preventing the activation of human stellate cells and promotes recovery from liver fibrosis in mice. Mol Ther. 24(10): 1848-59. 2016

### Summary of Invention

### Technical Problem

Direct administration to the portal vein is generally considered to be effective in order to administer a drug for treatment of the liver at a high concentration while suppressing deactivation. In the above-described report in which mice were used, the drug was administered to the caudal vein. It can be said that such an administration method is effective because the caudal vein can be easily punctured through the body surface and is in direct communication with the portal vein. However, humans have no blood vessels corresponding to the mouse caudal vein, and therefore, administering a drug through puncture of the portal vein inside the liver while checking using an abdominal ultrasonography is conceivable as a method that exhibits similar effects, for example. However, this method requires high skill and puts an extremely large burden on the patient, and therefore, it is not practical to put this method to practical use. Alternatively, administration through a common intravenous injection or subcutaneous injection is conceivable. However, in the case of administration through an intravenous injection, the drug reaches the liver via the lung, the digestive organs, and the like and may thus spread through the entire body. Also, the drug goes through the capillary vessels and may thus be deactivated. Accordingly, it is necessary to, for example, use the drug together with ethylene glycol, or use the drug in the form of a liposome formulation in which nucleic acid is encapsulated. Moreover, in the case of administration through a subcutaneous injection, the drug needs to be absorbed into the blood vessel from under the skin. Even when the drug is absorbed into the blood vessel, the same problem as in the case of intravenous injection also arises thereafter. Although miRNA-29a has been reported to be useful, when actual administration to a human is taken into consideration, the administration method includes a complex step of preparing a formulation and puts a large administration burden on the patient, for example, as described above. Accordingly, a method that puts a smaller burden on the patient is desired for treatment of liver fibrosis with a nucleic acid medicine.

Therefore, it is an object of the present invention is to provide a novel drug capable of inhibiting liver fibrosis while reducing the burden on the patient.

### Solution to Problem

To achieve the above-mentioned object, an miR-29a-like nucleic acid reagent according to the present invention includes nucleic acid having a base sequence of Sequence ID No. 1 that includes DNA and RNA and in which at least one nucleotide residue is a modified residue.

### Sequence ID No. 1: uagcaccaggctgucc

A liver fibrosis inhibitor according to the present invention includes the miR-29a-like nucleic acid reagent according to the present invention.

A pharmaceutical composition according to the present invention includes the miR-29a-like nucleic acid reagent according to the present invention.

A method for inhibiting liver fibrosis according to the present invention includes adding the miR-29a-like nucleic acid reagent according to the present invention.

### Advantageous Effects of Invention

When a drug is administered to a patient, oral administration is a method that puts a smaller burden on the patient. However, a nucleic acid medicine is decomposed, for example, by an enzyme or acid in the digestive organs, thus making oral administration difficult. As a result of extensive research conducted to address this, the inventors of the present invention found a novel nucleic acid that has a function higher than or equal to that of miR-29a and is stable enough to resist decomposition by an enzyme or the like even in the inner-body environment after oral administration. Accordingly, for example, the miR-29a-like nucleic acid reagent according to the present invention can be an alternative to miR-29a, and can thus be used for inhibition of liver fibrosis and treatment of a liver disease as well as for oral administration when administered.

### Brief Description of Drawings

[FIG. 1] FIG. 1 is an electrophoretic photograph illustrating the stability of an miR-29a-like nucleic acid reagent.
[FIG. 2] FIG. 2 is a graph illustrating fibrosis inhibition caused by the administration of the miR-29a-like nucleic acid reagent to liver fibrosis model mice.

### Description of Embodiments

The terms as used herein can be used in the general senses in the art unless otherwise stated.

### (1) miR-29a-Like Nucleic Acid Reagent

As described above, the miR-29a-like nucleic acid reagent according to the present invention is characterized in that it includes nucleic acid (also referred to as a "nucleic acid molecule according to the present invention" hereinafter) having a base sequence of Sequence ID No. 1 that includes DNA and RNA and in which at least one nucleotide residue is a modified residue. In Sequence ID No. 1, a region between position 1 and position 8 at the 5'-end corresponds to a seed sequence of miR-29a.

### Sequence ID No. 1: uagcaccaggctgucc

The modified residue may be, for example, a nucleotide residue that includes a modified base, or a nucleotide residue that includes a modified sugar residue.

There is no particular limitation on the DNA region and the RNA region in the base sequence. For example, the region between position 3 and position 13 or the region between position 4 and position 13 is the RNA region, and the region other than the RNA region is the DNA region.

In the base sequence, the RNA region is preferably an unmodified RNA region, and the DNA region other than the RNA region is a modified DNA region.

There is no particular limitation on the type of modification in the base sequence. In the case where the modification target is a base, examples of the modification include modifications with a halogen (halogenation). Examples of the halogenation include fluorination, chlorination, and bromination, and fluorination is preferable. Also, in the case where the modification target is a base, examples of the modification further include modifications with a methyl group (methylation) in addition to the modifications above. In the case where a uracil (u) in Sequence ID No. 1 is modified with a methyl group, a methylated uracil is a thymine.

In the case where the modification target is a sugar residue, examples of the modification include modifications with a LNA (Locked Nucleic Acid). The modifications with a LNA are, for example, modifications for forming a cross-linked nucleotide by cross-linking, via a methylene, the oxygen atom at position 2' and the carbon atom at position 4 in a sugar residue of a nucleotide. In addition to this, examples of the modified nucleotide residue further include a PNA (peptide nucleic acid) and an ENA (2'-O,4'-C-Ethylenebridged Nucleic Acid).

In the base sequence, for example, at least one uracil is a modified uracil, and it is preferable that at least one uracil in the DNA region is a modified uracil. A specific example of the modified uracil is a methyluracil obtained through methylation at position 5. As described above, a methyluracil is also referred to as a "thymine".

In the base sequence, for example, at least one cytosine in the DNA region is a modified cytosine. In the DNA region, all the cytosines may be modified cytosines, or one or two of the cytosines may be modified cytosines. It is preferable that the modified cytosine is a methylcytosine obtained through methylation, for example.

In the DNA region in the base sequence, for example, at least one nucleotide residue is a modified residue with a modified sugar residue, and specifically an LNA modified residue, for example. There is no particular limitation on the number of the modified residues with a modified sugar residue in the base sequence, and the number thereof is, for example, 1 to 6 residues, 1 to 3 residues, 2 residues, or 1 residue.

In the DNA region in the base sequence, at least one base is a halogen base obtained through halogenation.

In the base sequence, at least one uracil is a methyluracil, for example.

Specific examples of the base sequence that includes DNA and RNA and in which at least one nucleotide residue is a modified residue are shown below. That is to say, specific examples of the base sequence include base sequences of Sequence ID Nos. 2, 3, and 4. In these base sequences, for example, regions represented by uppercase letters are DNA regions, and regions represented by lowercase letters are RNA regions.
Sequence ID No. 2 (#2d): UAgcaccaggctgUCC
Sequence ID No. 3 (#2g): TAgcaccaggctgTCC
Sequence ID No. 4 (#GC): TAGcaccaggctgTCC

Specific examples of modifications in the base sequences of Sequence ID Nos. 2, 3, and 4 are shown below. That is to say, in these base sequences, for example, the underlined bases are modified bases obtained through modification with a halogen, the nucleotide residues that include a base surrounded by a rectangle are LNA modified residues, and m5C represents a methylated cytosine obtained through methylation at position 5.

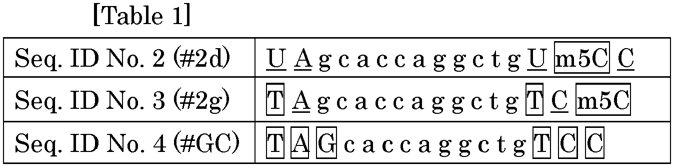

In the miR-29a-like nucleic acid reagent according to the present invention, the nucleic acid molecule may be a molecule having a sequence obtained by further coupling an additional sequence to one or both of the 5'-end and the 3'-end of the 16-base sequence. There is no particular limitation on the length of the additional sequence, and the length thereof is, for example, 1 to 3 bases, 1 or 2 bases, or 1 base.

There is no particular limitation on the application of the miR-29a-like nucleic acid reagent according to the present invention. As described above, the miR-29a-like nucleic acid reagent has a function equal to that of miR-29a and can thus be used as an alternative to miR-29a.

For example, the miR-29a-like nucleic acid reagent according to the present invention may include only the nucleic acid molecule, or may include other additives in addition to the nucleic acid molecule. There is no particular limitation on the additives, and examples thereof include a solvent, a vehicle, a carrier for supporting the nucleic acid molecule, a binding substance for a target cell, a condensing agent, and a fusing agent.

There is no particular limitation on a method of using the miR-29a-like nucleic acid reagent according to the present invention, and reference can be made to the following descriptions regarding the liver fibrosis inhibitor and the pharmaceutical composition according to the present invention.

### (2) Liver Fibrosis Inhibitor

As described above, the liver fibrosis inhibitor according to the present invention is characterized in that it includes the miR-29a-like nucleic acid reagent according to the present invention. The liver fibrosis inhibitor according to the present invention is characterized in that it includes the miR-29a-like nucleic acid reagent according to the present invention, and there is no limitation on other configurations and conditions.

For example, the liver fibrosis inhibitor according to the present invention may include only an active ingredient for inhibiting liver fibrosis, or may include the active ingredient and other additives. For example, the miR-29a-like nucleic acid reagent according to the present invention may be included as the only active ingredient, or another active ingredient may be included in addition to the miR-29a-like nucleic acid reagent. There is no particular limitation on the other active ingredient, and miR-29a may be used together, for example.

There is no particular limitation on the additives, and examples thereof include a solvent, a vehicle, a carrier for the miR-29a-like nucleic acid reagent, a binding substance for a target cell, a condensing agent, and a fusing agent. Moreover, the liver fibrosis inhibitor according to the present invention can also be used, for example, without liposome. The liposome may be used together with nucleic acid.

There is no particular limitation on the target to which the liver fibrosis inhibitor according to the present invention is to be added, and examples thereof include hepatocytes, liver tissues, and living organisms. The hepatocytes may be, for example, those collected from a living body, or those from a cell line.

There is no particular limitation on the sources of the cells and the tissues and the types of the living organisms, and examples thereof include humans and non-human mammals. Examples of the non-human mammals include cats, dogs, pigs, cows, sheep, monkeys, and bats. The liver fibrosis inhibitor according to the present invention may be added in vivo or in vitro depending on the addition target.

When the liver fibrosis inhibitor according to the present invention is added (administered) to a living organism, there is no particular limitation on the administration method, and examples thereof include oral administration and parenteral administration. As described above, the liver fibrosis inhibitor according to the present invention is very stable, for example, even when orally administered. Therefore, it is preferable that the liver fibrosis inhibitor is orally administered. Examples of the parenteral administration include an intravenous injection, a subcutaneous injection, local administration, intraperitoneal administration, and percutaneous administration.

When the liver fibrosis inhibitor according to the present invention is orally administered, there is no particular limitation on the administration conditions. For example, when administered to an adult male, the liver fibrosis inhibitor is administered at a dose per day of 1 to 50 mg in terms of the nucleic acid molecule according to the present invention once a day at an administration interval of 1 to 7 days.

### (3) Pharmaceutical Composition

As described above, a pharmaceutical composition according to the present invention is characterized in that it includes the miR-29a-like nucleic acid reagent according to the present invention. The pharmaceutical composition according to the present invention is characterized in that it includes the miR-29a-like nucleic acid reagent according to the present invention, and there is no limitation on other configurations and conditions. As for a method of using the pharmaceutical composition according to the present invention, reference can be made to the description regarding the liver fibrosis inhibitor according to the present invention.

The pharmaceutical composition according to the present invention can be used for, for example, diseases against which miR-29a may serve as an active ingredient. Examples of the target diseases include liver diseases, specifically chronic liver diseases, liver cirrhosis, and the like. In particular, the pharmaceutical composition can be applied to a state in which liver fibrosis is likely to be developed and a state in which liver fibrosis has been developed, irrespective of the causes of liver diseases, including, for example, liver disorders caused by infection of viruses (e.g., HBV, HCV, and the like), liver disorders caused by alcohol ingestion, fatty liver, non-alcoholic steatohepatitis, autoimmune hepatitis, primary biliary cholangitis, and the like.

Although there is no limitation on the method of administering the pharmaceutical composition according to the present invention, it is preferable that the pharmaceutical composition is orally administered, for example, as described above.

### (4) Method for Inhibiting Liver Fibrosis and Method for Treating Liver Disease

The method for inhibiting liver fibrosis according to the present invention is characterized in that it includes adding the miR-29a-like nucleic acid reagent according to the present invention, the liver fibrosis inhibitor according to the present invention, or the pharmaceutical composition according to the present invention. Moreover, the method for treating a liver disease according to the present invention is characterized in that it includes administering the miR-29a-like nucleic acid reagent according to the present invention, the liver fibrosis inhibitor according to the present invention, or the pharmaceutical composition according to the present invention. The present invention is characterized in that the miR-29a-like nucleic acid reagent according to the present invention, the liver fibrosis inhibitor according to the present invention, or the pharmaceutical composition according to the present invention is used, and there is no limitation on other steps and conditions. As for the method for inhibiting liver fibrosis and the method for treating a liver disease according to the present invention, reference can be made to the descriptions regarding the miR-29a-like nucleic acid reagent, the liver fibrosis inhibitor, and the pharmaceutical composition according to the present invention.

The addition targets are, for example, cells, tissues, or living organisms as described above, and examples of the living organisms include humans and the above-mentioned non-human animals. When addition (administration) to a target is performed in vivo as described above in the addition step, the administration method is preferably oral administration, for example.

### (5) Use of Nucleic Acid

Use according to the present invention is use of the nucleic acid molecule according to the present invention for inhibition of liver fibrosis or treatment of a liver disease.

Hereinafter, the present invention will be described in detail using examples and the like. However, the present invention is not limited to the examples and the like below.

### [Examples]

### (Example 1)

Three types of nucleic acid molecules (#2d, #2g, #GC) shown in Table 2 were used as the miR-29a-like nucleic acid reagent and were subjected to a stability test and a pathological test. Also, a nucleic acid molecule miR-29a (Gene Design Co., Ltd.) was used as a reference example.

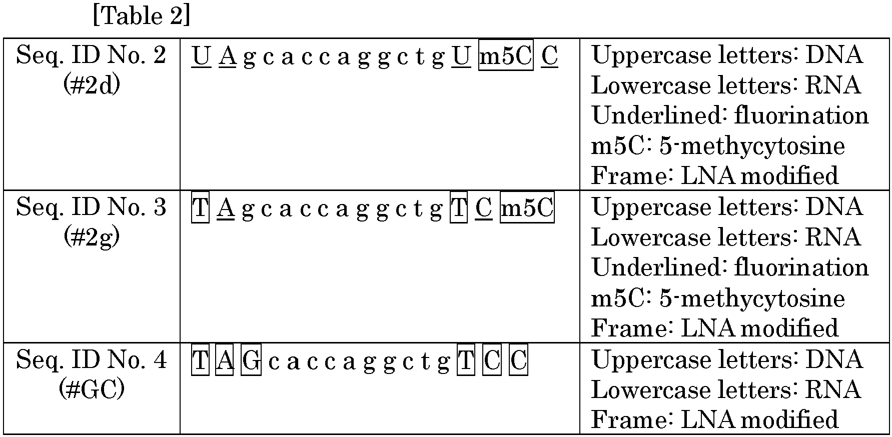

### (1) Stability Test

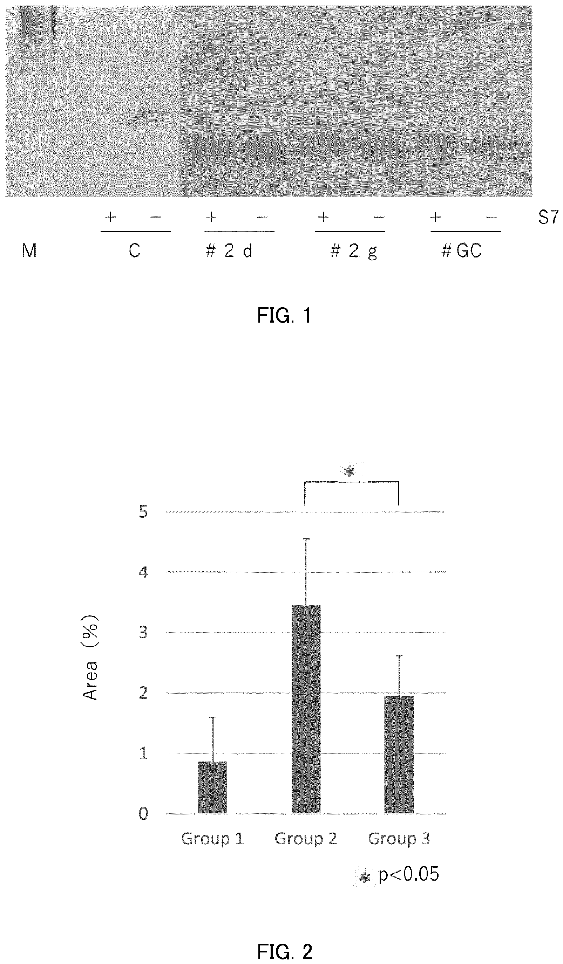

A commercially available reagent (trade name: Nuclease S7, Sigma-Aldrich) was used as an S7 nuclease. 1 µg of each of the nucleic acid molecule and 10 U of the S7 nuclease were mixed in a buffer solution for the S7 nuclease, the resultant mixture was incubated at 37°C for 10 minutes, and the reaction was ended by adding EDTA. Then, the reaction solution was subjected to polyacrylamide gel electrophoresis. Also, the nucleic acid molecules were treated in the same manner as described above, except that the enzyme was not added, and then electrophoresis was performed. FIG. 1 shows the results. FIG. 1 is a photograph of gel illustrating the results of electrophoresis. The lanes in FIG. 1 illustrate the results from a molecular-weight marker (M), miR-29a (control: C), #2d, #2g, and #GC, in the order from left to right. In addition, "+" indicates the result from the case where the enzyme was added, and "-" indicates the result from the case where the enzyme was not added. As shown in FIG. 1, the band corresponding to miR-29a disappeared due to the enzyme treatment, whereas the bands corresponding to #2d, #2g, and #GC remained even after the enzyme treatment. It was found from these results that enzymatic decomposition of the miR-29a-like nucleic acid reagent according to the present invention was inhibited.

### (2) Pathological Test

6-week-old mice were divided into 3 groups (n=7 per group): the first group was "control 1", the second group was "control 2", and the third group was "example group". Fibrosis in the liver was caused through administration of carbon tetrachloride. The following is the specific procedure.

Olive oil was intraperitoneally administered to the mice of the first group at a dose of 0.5 µl/g (weight) once a day every 3 days for 7 weeks. Olive oil containing carbon tetrachloride (FUJIFILM Wako Pure Chemical Corporation) was intraperitoneally administered to the mice of the second group at a dose of 0.5 µl/g (weight) once a day every 3 days for 7 weeks (16 times in total). The concentration of carbon tetrachloride in the olive oil containing carbon tetrachloride was 10% (w/v). The olive oil containing carbon tetrachloride (FUJIFILM Wako Pure Chemical Corporation) was intraperitoneally administered to the mice of the third group at a dose of 0.5 µl/g (weight) once a day every 3 days for 7 weeks (16 times in total). In addition, during the period from week 5 to week 7, a nucleic acid solution (solvent: pure water) containing #2d as the miR-29a-like nucleic acid reagent was orally administered at a dose of 200 µL (the content of #2d: 20 µg) once a day every 3 days. After the end of week 7, the liver was extracted from each mouse, and was subjected to fiber stain and HE stain. The ratio of a fiber-stained area in an HE-stained area was calculated and was defined as "fiber stain positive area (%)". Significant differences between the fiber stain positive areas were determined by Student t-test (* p<0.05).

FIG. 2 shows the results. FIG. 2 is a graph illustrating the fiber stain positive areas (%) of the groups. As shown in FIG. 2, the fiber stain positive area significantly increased in the second group to which carbon tetrachloride had been administered, compared with the first group to which carbon tetrachloride had not been administered. It was confirmed from this result that fibrosis was caused by the administration of carbon tetrachloride. The fiber stain positive area significantly decreased in the third group to which the miR-29a-like nucleic acid reagent had been added in addition to carbon tetrachloride, compared with the second group. It was found from this result that the administration of the miR-29a-like nucleic acid reagent effectively inhibited fibrosis. Liposome, which is commonly used in administration of a nucleic acid molecule, was not used together in this oral administration, and it was thus found that the miR-29a-like nucleic acid reagent is effectively absorbed into the body without using liposome together and can inhibit fibrosis in the liver.

Although the present invention has been described above with reference to the embodiments and the examples, the present invention is not limited to the embodiments above. Various changes that may be understood by those skilled in the art can be made to the configuration and details of the present invention without departing from the scope of the present invention.

This application claims the benefit of priority from Japanese Patent Application No. 2020-187521, filed on November 10, 2020, the entire contents of which are incorporated herein by reference.

### Industrial Applicability

The miR-29a-like nucleic acid reagent according to the present invention can be an alternative to miR-29a, for example, and can thus be used for inhibition of liver fibrosis and treatment of a liver disease as well as for oral administration when administered.

### [Sequence Listing]

Sequence listing ¥TMU20014WO_ST25.txt

## Claims

1. An miR-29a-like nucleic acid reagent comprising nucleic acid having a base sequence of Sequence ID No. 1 that includes DNA and RNA and in which at least one nucleotide residue is a modified residue:
Sequence ID No. 1: uagcaccaggctgucc.

2. The miR-29a-like nucleic acid reagent according to claim 1, wherein the modified residue is a nucleotide residue that includes a modified base, or a nucleotide residue that includes a modified sugar residue.

3. The miR-29a-like nucleic acid reagent according to claim 1 or 2, wherein, in the base sequence, a region between position 3 and position 13 or a region between position 4 and position 13 is an RNA region, and a region other than the RNA region is a DNA region.

4. The miR-29a-like nucleic acid reagent according to claim 3, wherein the region between position 3 and position 13 or the region between position 4 and position 13 is an unmodified RNA region, and a region other than the unmodified RNA region is a modified DNA region.

5. The miR-29a-like nucleic acid reagent according to any one of claims 1 to 4, wherein at least one uracil in the base sequence is a methyluracil obtained through methylation at position 5.

6. The miR-29a-like nucleic acid reagent according to any one of claims 3 to 5, wherein at least one cytosine in DNA region in the base sequence is a methylcytosine obtained through methylation.

7. The miR-29a-like nucleic acid reagent according to any one of claims 3 to 6, wherein at least one nucleotide residue in the DNA region in the base sequence is an LNA modified residue.

8. The miR-29a-like nucleic acid reagent according to any one of claims 3 to 7, wherein at least one base in the DNA region in the base sequence is a halogen base obtained through halogenation.

9. The miR-29a-like nucleic acid reagent according to any one of claims 1 to 8, wherein at least one uracil in the base sequence is a methyluracil.

10. The miR-29a-like nucleic acid reagent according to any one of claims 1 to 9, wherein the base sequence is a base sequence of Sequence ID No. 2, 3, or 4, where a region represented by uppercase letters is a DNA region and a region represented by lowercase letters is an RNA region:
Sequence ID No. 2 (#2d): UAgcaccaggctgUCC;
Sequence ID No. 3 (#2g): TAgcaccaggctgTCC;
Sequence ID No. 4 (#GC): TAGcaccaggctgTCC.

11. The miR-29a-like nucleic acid reagent according to claim 10, wherein, in the base sequences of Sequence ID Nos. 2, 3, and 4, underlined bases are modified bases obtained through modification with a halogen, nucleotide residues that include a base surrounded by a rectangle are LNA modified residues, and m5C represents a methylated cytosine obtained through methylation at position 5

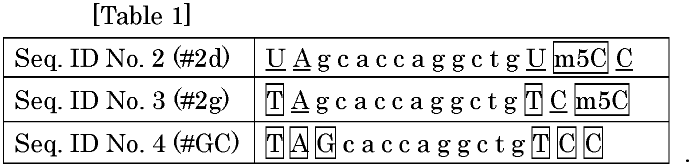

12. A liver fibrosis inhibitor comprising the miR-29a-like nucleic acid reagent according to any one of claims 1 to 11.

13. A pharmaceutical composition comprising the miR-29a-like nucleic acid reagent according to any one of claims 1 to 11.

14. The pharmaceutical composition according to claim 13, which is for a liver disease.

15. The pharmaceutical composition according to claim 13 or 14, which is for oral administration.

16. A method for inhibiting liver fibrosis comprising adding the miR-29alike nucleic acid reagent according to any one of claims 1 to 12.

17. A method for treating a liver disease comprising administering the miR-29a-like nucleic acid reagent according to any one of claims 1 to 12.

18. Nucleic acid having a base sequence of Sequence ID No. 1 that includes DNA and RNA and in which at least one nucleotide residue is a modified residue, the nucleic acid being used to inhibit liver fibrosis:
Sequence ID No. 1: uagcaccaggctgucc.
